# EUROPEAN PATENT SPECIFICATION

(11) **EP 2 805 693 B1**
(45) Date of publication and mention of the grant of the patent: **16.09.2015**
(21) Application number: 14181464.0
(22) Date of filing: 22.11.2010
(51) Int. Cl.: A61F 2/06, A61M 25/06, A61F 2/24, A61B 17/00, A61M 25/00, A61F 2/958

(54) **Vascular introducers having an expandable section**
Gefässeinführungsinstrumente mit expandierbarem Teil
Dispositifs d'introduction vasculaires dotés d'une section dilatable

(30) Priority: 03.02.2010 US 300999 P
(43) Date of publication of application: 26.11.2014
(62) Divisional of application: 10845405.9
(73) Proprietor: CircuLite, Inc., Teaneck, NJ 07666 (US)
(72) Inventor: Farnan, Robert C, Fort Lauderdale, Florida 33308 (US)
(74) Representative: Findlay, Alice Rosemary

(56) References cited:
- WO-A1-98/06448
- US-A- 6 090 072
- US-A1- 2001 012 946
- US-A1- 2002 169 495
- US-A1- 2008 200 943
- US-A1- 2008 243 081

## Description

### Technical Field

The present invention is directed generally to devices used to access the vascular network of a patient. More specifically, the present invention is related to vascular introducers and introducer assemblies.

### Background

The use of catheter-based procedures has reduced the invasive nature of some surgical procedures. For example, previous procedures for implanting surgical devices into the heart or for repairing heart tissue previously required at minimum a thoracotomy, i.e., the opening of the thoracic cavity between successive ribs to expose the internal organs. More typically, these procedures required cardiac surgery, generally known as open-heart surgery, where the sternum is cut and split to expose the internal organs. Once the thoracic cavity is accessed, the surgeon must enter the pleural space and puncture both the pericardium and the myocardial wall. There are great risks and an extensive recovery time associated with the invasive nature of the implantation surgery. As such, some patients with severe symptoms are not healthy enough for surgery to receive a circulatory assist system. By implementing percutaneous methods via catheter, the invasiveness of the procedure may be diminished and in return a greater number of patients can receive the surgical benefit.

During the catheter-based procedures, the catheter is inserted into the vascular network by way of an introducer that provides several advantages. One advantage is that the introducer can maintain a fluidic access site into vascular structure while limiting the amount of bleed out. Another advantage is the ability of the introducer to dilate the access site that would otherwise collapse onto the catheter and resist movement of the catheter relative to the vessel.

Despite the use of the introducer, there remain some areas for improvement. For example, the rigid construction of conventional introducers has limited use to locations of the vascular network having a vessel that is relatively straight. It would be of great benefit to be able to insert the introducer into the vessel near a bifurcation joint with other vessels so that surgical instruments can smoothly traverse the joint. Another area for improvement includes the ability to use large diameter introducers to accommodate larger surgical instruments, without requiring a large diameter access site in the vessel wall. Yet another area for improvement would include introducers that are capable of navigating through a curved vasculature without kinking along the length of the introducer or straightening the vasculature.

US 2001/0012946 discloses an introducer configured to permit vascular access to at least one surgical tool during a percutaneous procedure, the introducer comprising a sheath configured to be inserted into a vasculature of a patient, the sheath having at least one expandible section. US 2008/243081 also discloses an introducer comprising a sheath configured to be inserted into a vasculature of a patient, the sheath having at least one expandible section.

### Summary

The invention provides an introducer which is characterized in that the sheath is sufficiently flexible to span a junction between a first blood vessel and a second blood vessel, and in that the introducer further comprises a hub on a proximal end of the sheath and having a hemostasis valve disposed thereon, the hub being configured to remain external to the vasculature and the hemostasis valve being configured to limit access through the lumen of the sheath.

The sheath may be expandable from a first profile to a second profile. The sheath in the first profile is configured for directing the introducer into the vasculature of a patient while the sheath in the second profile is configured to receive a catheter. Appropriate catheters may be configured to deliver a surgical tool, to move blood, or may be the surgical tool. The sheath may include a balloon-expandable section and a self-expandable section proximal to the balloon-expandable section. The balloon-expandable section may include a plurality of balloon-expandable struts that may be constructed from a deformable metallic material. The self-expandable section may include a plurality of self-expandable struts that may be constructed from a superelastic material.

The hemostasis may be a slit with a membrane or an iris. The flexibility of the sheath may increase distally.

In some embodiments, at least a portion of the sheath is comprised of a self-expandable material. The sheath may include one or more markers comprised of a radiopaque material configured to enable in vivo visualization of the sheath. In one embodiment, an inner diameter surface of the sheath, an outer diameter of the sheath, or both is coated with a coating that is operable to lower the coefficient of the surface..

In another illustrative embodiment, an introducer assembly includes the introducer having an expandable profile and a dilator that extends through the lumen of the introducer. The dilator includes an inflation member that is positioned within the balloon-expandable section of the sheath of the introducer.

There is disclosed a method of using the introducer assembly is provided. The method includes directing the introducer assembly into the lumen of the vasculature of the patient. The inflation member is then inflated, causing the diameter of the sheath of the introducer to increase.

Other illustrative embodiments are directed to a percutaneous surgical system comprising a delivery device, a catheter, and the introducer having an expandable sheath. The introducer is configured to receive the delivery device for insertion into the vasculature of a patient. The sheath of the introducer is configured to expand, allowing the delivery device to be retracted and replaced with the catheter. The catheter is operable to effectuate a surgical benefit.

Further disclosed is a method of performing a catheter-based procedure with an introducer having an expandable sheath. The method includes inserting the introducer into the vasculature of the patient and expanding the expandable sheath of the introducer. The expandable sheath, once expanded, may receive a catheter.

### Brief Description of the Drawings

FIG. 1 is a diagrammatic view of a portion of the vascular network of the upper thorax with a guide-wire and one exemplary embodiment of an introducer assembly directed into the vascular network.
FIG. 2A is a disassembled, perspective side view of one exemplary embodiment of the introducer assembly including an introducer and a dilator, shown in cross-section.
FIG. 2B is a perspective side view, in partial cross-section, illustrating the assembled introducer assembly in a collapsed state.
FIG. 2C is a perspective side view, in partial cross-section, illustrating the assembled introducer assembly in an expanded state.
FIG. 3 is an enlarged view of an alternate embodiment of a hub of the introducer.
FIGS. 4A-4C are diagrammatic views, in partial cross-section, illustrating successive steps of one exemplary procedure for directing the exemplary embodiment of the introducer assembly of FIG. 2A into a vein.
FIG. 4D is a diagrammatic view, in partial cross-section, illustrating the expanded introducer within the vein with the dilator removed.
FIG. 4E is a diagrammatic view illustrating the expanded introducer within the vein with a cannula directed through the introducer and into the vascular network, shown in partial cross-section.

### Detailed Description

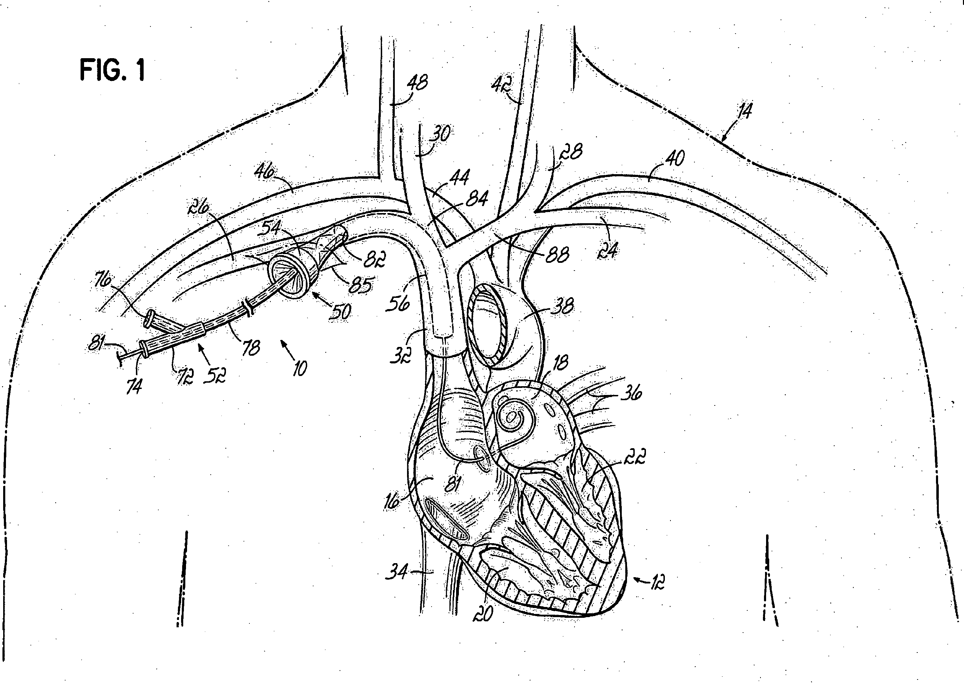

FIG. 1 illustrates an introducer assembly 10 according to one embodiment of the invention directed into the vascular network. For illustrative purposes, certain anatomy is shown including the heart 12 of a patient 14 having a right atrium 16, a left atrium 18, a right ventricle 20, and a left ventricle 22. Blood from the left and right subclavian veins 24, 26 and the left and right jugular veins 28, 30 enters the right atrium 16 through the superior vena cava 32 while blood from the lower parts of the body enters the right atrium 16 through the inferior vena cava 34. The blood is pumped from the right atrium 16, to the right ventricle 20, and to the lungs (not shown) to be oxygenated. Blood returning from the lungs enters the left atrium 18 via pulmonary veins 36 and is then pumped into the left ventricle 22. Blood leaving the left ventricle 22 enters the aorta 38 and flows into the left subclavian artery 40, the left common carotid 42, and the innominate artery 44 including the right subclavian artery 46 and the right common carotid 48.

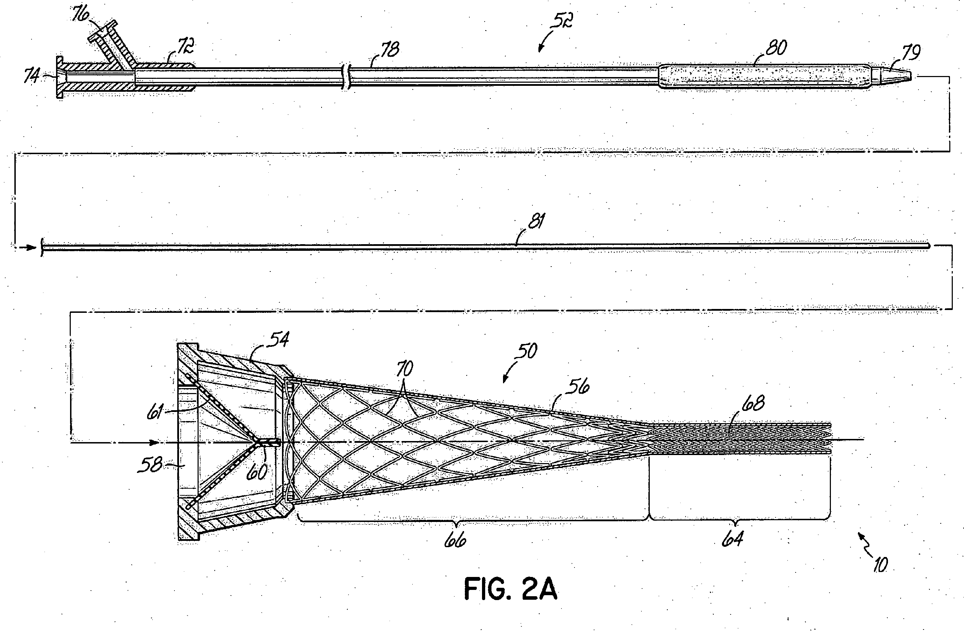

Turning now to FIG. 2A where the details of the introducer assembly 10, including an introducer 50 and a dilator 52, are shown with greater detail. The introducer 50 includes a hub 54 that remains external to the appropriate vascular structure (illustrated in FIG. 1 as the right subclavian vein 26) and a sheath 56 that will extend into the lumen of the right subclavian vein 26. It will be understood by those that are of ordinary skill in the art that use of the introducer assembly 10 is not limited to the right subclavian vein 26, but instead may be used with other appropriate vascular structures where percutaneous access is desired.

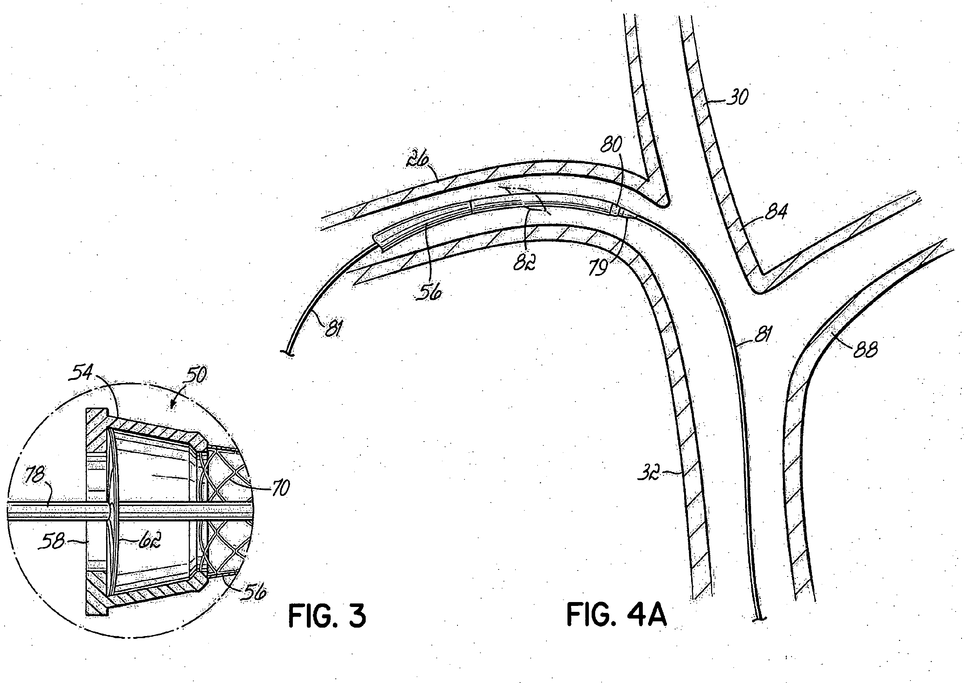

The hub 54 of the introducer 50 is coupled to a proximal end of the sheath 56 by a chemical adhesive, a thermal welding process, or a melting process. The hub 54 includes a port 58 that allows passage of the dilator 52 or other surgical instruments that will be used in the following procedure and in a manner that is described in greater detail below. Distal to the port 58 is a hemostatic valve that is configured to allow for the passage of a surgical device, including the dilator 52, while maintaining a hemostatic seal. Various structures for hemostatic valves are known to those of ordinary skill in the art, and may include a slit 60 within a membrane 61 (for example, an elastomeric or thermoplastic elastomeric material) or an iris 62 (FIG. 3) having moveable sections to actuate the seal.

In some embodiments, though not specifically shown herein, the hub 54 may also include additional seals, such as an O-ring, to further enhance the sealing against the dilator 52 or any other subsequently introduced surgical device.

Referring still to FIG. 2A, the sheath 56 of the introducer 50 includes a distal balloon-expandable section 64 and a proximal self-expandable section 66. The distal balloon-expandable section 64 may include a multi-layer construction including: an inner layer formed from an expandable, low coefficient of friction polymeric material (such as ePTFE, nylon, or polyethylene), an outer layer may be constructed from a polymeric material (such as Nylon, polyurethane, or polyethylene) having a low coefficient of friction and a low durometer, and balloon-expandable struts 68 between the inner and outer layers. The balloon-expandable struts 68 may be made of a deformable metallic material, such as stainless steel, nickel cobalt, or chromium cobalt, and are constructed in a manner that is similar to a conventional balloon-expandable stent. The distal balloon-expandable section 64 may range in length from about 5 mm to about 5 cm as necessary to achieve the particular surgical procedure.

The proximal self-expandable section 66 may also be a multi-layer construction. In some embodiments, the proximal self-expandable section 66 may be constructed from the same materials as the distal balloon-expandable section 64; however, this is not required. The layers of the proximal self-expandable section 66 encapsulate self-expanding struts 70 constructed from a superelastic-shape memory material, such as nickel titanium, that may be constructed in a manner that is similar to conventional self-expandable stents. The proximal self-expandable section 66 may range in length from about 8 cm to about 25 cm as desired by the physician for a particular surgical procedure.

It would be understood that the inner layer, the outer layer, or a combination thereof of the distal balloon-expandable section 64 and the proximal self-expandable section 66 should be constructed from the same or similar material to facilitate bonding. In some embodiments, the material of the inner layer, the outer layer, or both may extend the full length of the sheath 56 as a continuous, unitary structure without joints. The inner diameter surface, the outer diameter surface, or both, of the sheath may further include a coating that lowers the coefficient of friction of the sheath. Suitable coatings may include hydrophilic coatings, such as a silicone lubricant or a urethane-based hydromer; however, other coatings may also be used.

When the outer layer is constructed of separate materials, one of ordinary skill in the art would understand that the rigidity of the proximal portions of the outer layer should be greater than the rigidity of the distal portions of the outer layer to create a device that may be pushed into the vasculature. Further, it would also be understood that the cross-sections of the balloon-expandable struts 68 and the self-expanding struts 70 may be welded together, or otherwise joined in a known manner, such that expansion of the distal balloon-expandable section 64 translates to an expansion of the proximal self-expandable section 66.

FIG. 2A further illustrates one suitable dilator 52 that may be used for directing the introducer assembly 10 into the appropriate vessel. The dilator 52 may be a conventional balloon catheter, such as those manufactured by Boston Scientific, Natick, MA, or a custom dilator such as the removable dilator having an elongated taper and as described in detail in Appl. Ser. No. 61/163,931. Generally, the dilator 52 will include a proximal hub 72 (illustrated here as including a main port 74 and a side port 76), a shaft 78 having a distal tip 79, and a distally positioned inflation member, such as a balloon 80 that is configured to perform as an obtuator. As illustrated, the main port 74 allows entry and passage of a guide-wire 81 while the side port 76 permits fluidic access for inflation of the balloon 80, as described in detail below. The side port 76 may include a stop cock (not shown) for altering and maintaining the interstitial pressure to inflate or deflate the inflatable balloon 80. The dilator 52 will generally include a common lumen extending the full length for receiving and moving relative to the guide-wire 81. It would be understood that a hemostatic y-connector (not shown) may extend proximally from the main port 74 to allow flushing of the lumen and to prevent back bleeding. It would be further understood that while the dilator 52 is illustrated generally herein as a balloon catheter, other obtuators or similar apparatii may alternatively be used.

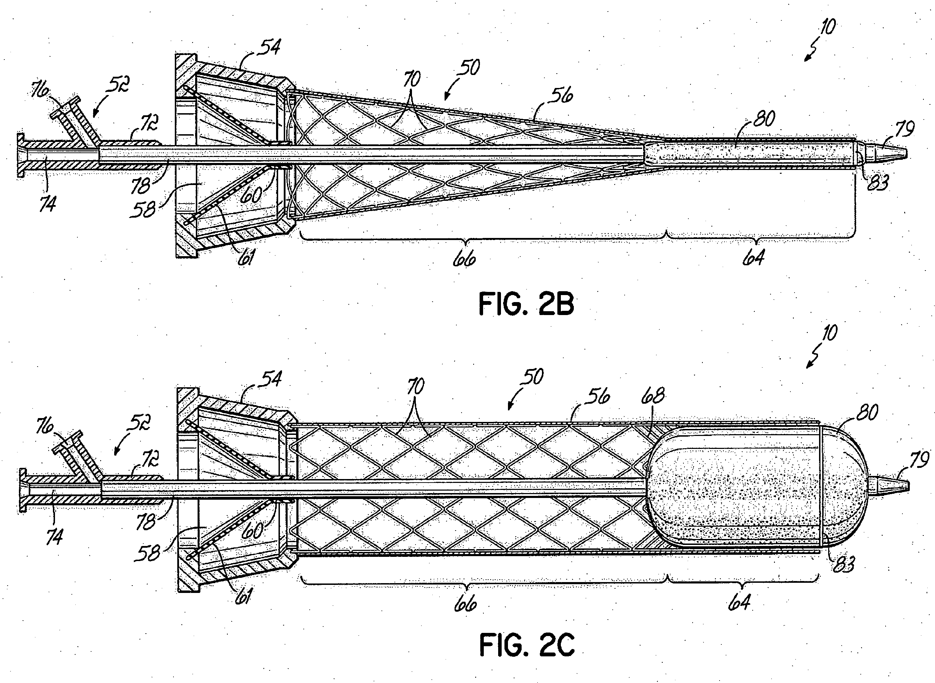

FIG. 2B illustrates the introducer assembly 10 ready for use where the dilator 52 is directed into the hub 54 of the introducer 50 and through the sheath 56 until the balloon 80, in its deflated state, is situated within the distal balloon-expandable section 64. As shown, the balloon 80 may include a marker 83 that may be used to align with the distal edge of the sheath 56 to aid in vivo visualization. The marker 83 may be constructed from a dense metallic material, such as gold (Au) or platinum (Pt), or from a polymeric material embedded with a dense powder, such as tungsten (W), that will allow the physician to visualize the introducer assembly 10 with non-invasive devices, such as X-ray, real-time fluoroscopy, or intracardiac echocardiograph. While only one marker 83 is shown, it would be understood that multiple markers could be used. With the balloon 80 properly positioned, the distal balloon-expandable section 64 is crimped or compressed onto the balloon 80 in a manner that is generally known and conventionally used with balloon-expandable stents. Once in the compressed state for delivery, the diameter of the balloon-expandable section 64 may be less than about 1 mm or about 3 mm, depending on the wall thickness or the manner of construction.

FIG. 2C illustrates the introducer assembly 10 in the expanded state caused by the inflation of the balloon 80, which is described in greater detail below.

With the details of the present embodiment described, one manner of using the introducer assembly 10 is shown in detail with reference to FIGS. 1 and 4A-4D.

FIG. 4A illustrates an incision 82 that is made in the wall of a superficial vessel, which for illustrative purposes only is shown to be in the wall of the right subclavian vein 26 near its juncture with the right jugular vein 30 and the right innominate vein 84. Access to the right subclavian vein 26 may be made by way of a vascular access site 85 (FIG. 1) located proximal the incision 82 by a scalpel or by puncturing the wall of the vessel with a guide-wire 81, or alternatively, a needle (not shown) that is then followed with the guide-wire 81. The guide-wire 81 is advanced through the incision 82, through the right innominate vein 84, past the left innominate vein 88, down the superior vena cava 32, and into the right atrium 16 (FIG. 1).

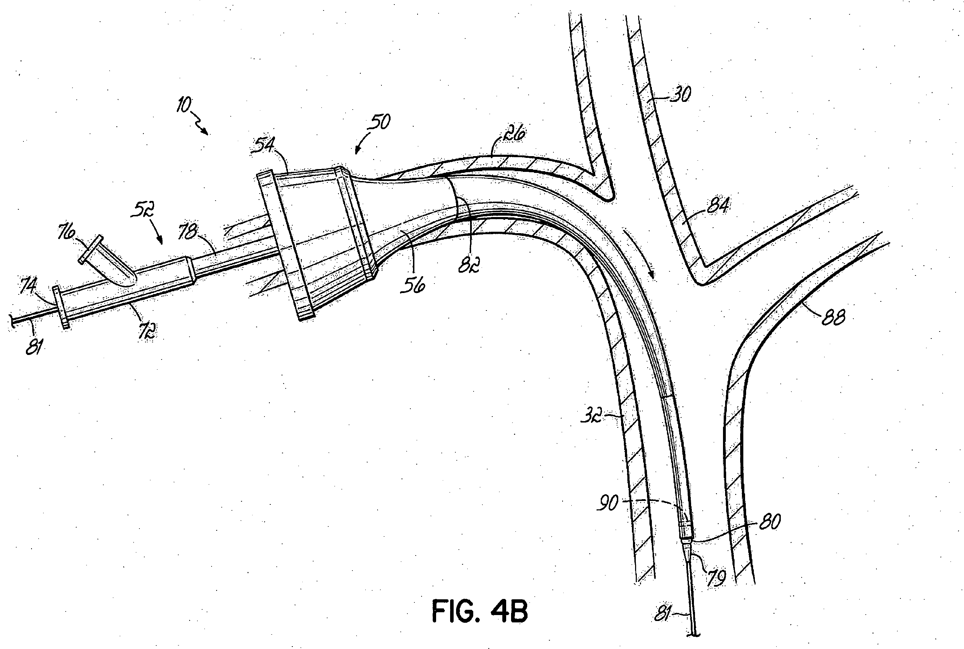

The physician then back-loads the introducer assembly 10 over the guide-wire 81, through the incision 82, and into the lumen of the right subclavian vein 26. Directing the introducer assembly 10 continues until the sheath 56 is positioned at a desired location, illustrated in FIG. 4B with the distal end of the assembly 10 being within the lumen of the superior vena cava 32.

While the balloon-expandable and self-expandable struts 68, 70 (FIG. 2A) will inherently possess some degree of radiopacity, the sheath 56 of the introducer 50 may include one or more markers 90 constructed from a material similar to those described above with reference to the marker 83 (FIG. 2B) of the balloon 80. It should be appreciated that the marker 90 should not completely surround the sheath 56 as a unitary structure as this may restrict operation of the balloon-expandable struts 68 (FIG. 2A). Instead, the markers 90 may be a dot or other appropriate shape.

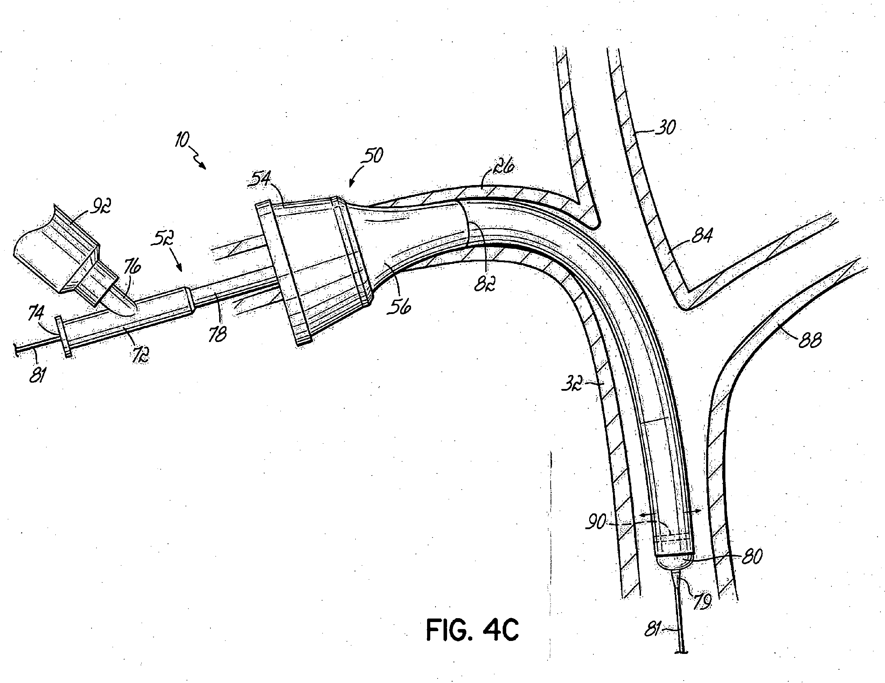

The physician may begin inflating the balloon 80 of the dilator 52 as shown in FIG. 4C. Accordingly, a syringe 92 having a stop cock (not shown) may be coupled to the side port 76 of the hub 72 and is used to direct an inflation fluid, such as saline, into the balloon 80. With sufficient inflation fluid, the interstitial fluidic pressure within the balloon 80 increases and resultantly expands the balloon 80. As the balloon 80 continues to radially expand, the outer surface of the balloon 80 adjacent the inner surface of the distal balloon-expandable section 64 (FIG. 2A) causes the distal balloon-expandable section 64 (FIG. 2A) of the sheath 56 to likewise expand. Inflation continues until a desired diameter of the sheath 56 is achieved, which may be observed in vivo, though generally fully inflating the balloon 80 results in fully dilating the sheath 56. Because the balloon-expandable struts 68 (FIG. 2A) and self-expandable struts 68 (FIG. 2A) are physically coupled, expansion of the distal balloon-expandable section 64 (FIG. 2A) will also induce some expansion of the proximal self-expandable section 66 (FIG. 2A).

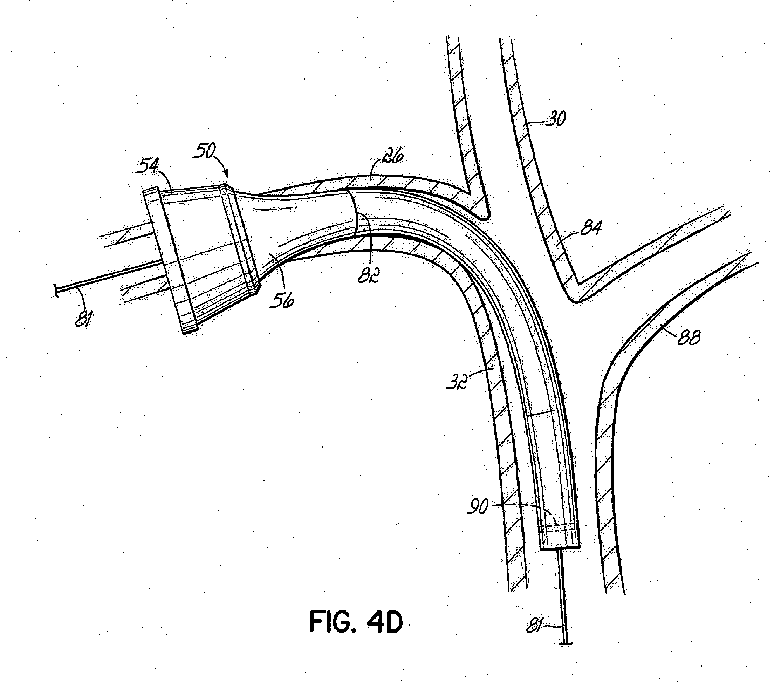

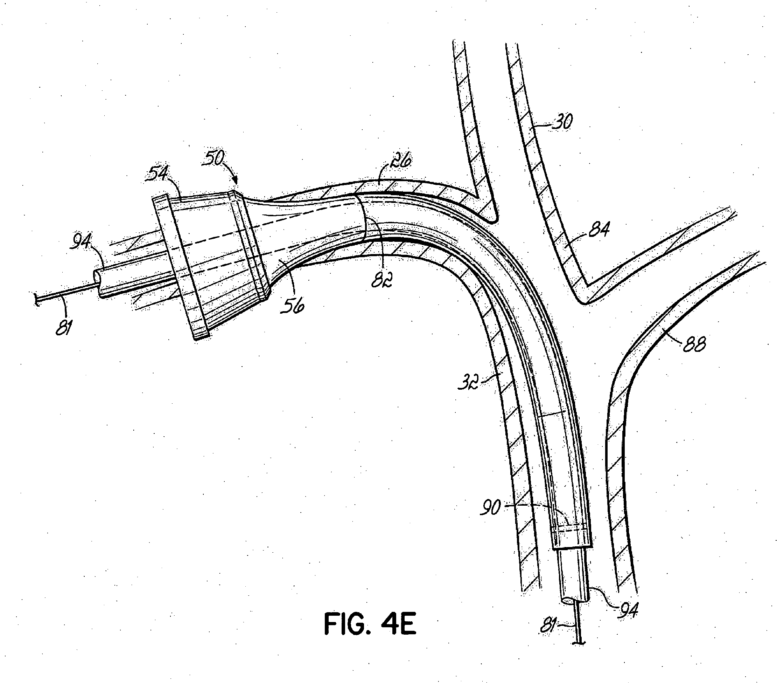

While the portion of the sheath 56 distal to the hub 72 is illustrated with a slight taper in FIGS. 4C, 4D, and 4E, it would be understood that a fully expanded sheath 56 would not necessarily include this taper.

With the distal balloon-expandable section 64 (FIG. 2A) of the sheath 56 fully expanded, the physician can deflate the balloon 80 by removing at least a portion of the inflation fluid and then retracting the dilator 52. As the physician retracts the dilator 52, the physician, at his discretion, may intermittently re-inflate the balloon 80 at various positions along the length of the proximal self-expandable section 66 (FIG. 2A) to ensure that it is fully expanded. The dilator 52 is then fully retracted from the introducer 50, as shown in FIG. 4D.

In some embodiments, the expansion of the sheath 56 may induce a decrease in the over-all length of the sheath 56 as the balloon-expandable struts 68 (FIG. 2A) change from the crimped to the expanded diameter and possible foreshortening in the self-expandable struts 70 (FIG. 2A); however, shortening of the sheath 56 or a lack thereof does not adversely affect the performance of the introducer 50, nor is it required.

As illustrated herein, the physician may advantageously position the sheath 56 in an area within the vessel where the diameter of the lumen exceeds to the desired final diameter of the introducer 50. This will ensure that neither the balloon-expandable nor the self-expanding struts 68, 70 (FIG. 2A) embed within the inner surface of the wall of the vessel. However, this is not necessarily required.

With the dilator 52 removed, the physician may then continue with a desired catheter-based procedure and direct a catheter 94 through the hemostatic valve and into the lumen of the superior vena cava 32, shown herein with the catheter 94 inserted into the vascular network via the introducer 50 and having a distal end of the catheter 94 residing within the right atrium 16 (FIG. 1). It would be readily appreciated that the catheter 94 may be any delivery tool, a catheter for moving blood, or another tool for the percutaneous procedure. The introducer 50 protects the walls of the right subclavian 26 and right innominate veins 84 while these devices are moved into the venous network. Additionally, the introducer 50 increases the ease by which these surgical devices enter the venous network by preventing the incision 82 from collapsing onto the surgical device and resisting movement of the same.

After the procedure is complete, the physician may retract the devices and guide-wire 81 from the introducer 50, and finally the introducer 50 from the right subclavian vein 26. The incision 82 and vascular access site 85 are then sutured or closed with a vascular closure device in a manner that would be known in the art.

While the present invention has been illustrated by a description of various preferred embodiments and while these embodiments have been described in some detail, additional advantages and modifications will readily appear to those skilled in the art. The various features of the invention may be used alone or in any combination depending on the needs and preferences of the user. This has been a description of the present invention, along with the preferred methods of practicing the present invention as currently known.

## Claims

1. An introducer (10) configured to permit vascular access to at least one surgical tool during a percutaneous-procedure, the introducer (10) comprising:
a sheath (56) having a proximal end, a distal end, and a lumen extending therebetween,
**characterized in that** the sheath (56) is sufficiently flexible to span a junction between a first blood vessel and a second blood vessel; and **in that** the introducer (10) further comprises a hub (54) on the proximal end of the sheath (56) and having a hemostasis valve (60, 61, 62) disposed therein, the hub (54) being configured to remain external to the vasculature and the hemostasis valve (60, 61, 62) being configured to limit access through the lumen of the sheath (56).

2. The introducer (10) of claim 1, wherein the hemostasis valve is a slit (60) within a membrane (61) or an iris (62).

3. The introducer (10) of claim 1, wherein the sheath (56) is expandable from a first profile configured for directing the introducer (10) into the vasculature to a second profile configured for receiving the surgical tool.

4. The introducer (10) of claim 1, wherein the flexibility of the sheath (56) increases distally.

5. The introducer (10) of claim 1, wherein at least a first portion (64) of the sheath (56) is comprised of a balloon-expandable material.

6. The introducer (10) of claim 5, wherein at least a second portion (66) of the sheath (56) is comprised of a self-expandable material, the second portion (66) being proximal to the first portion (64).

7. The introducer (10) of claim 6, wherein the first portion (64) of the sheath (56) includes a plurality of balloon-expandable struts (68) and the second portion (66) includes a plurality of self-expandable struts (70).

8. The introducer (10) of claim 7, wherein the sheath (56) includes an inner layer and an outer layer with the pluralities of balloon-expandable and self-expandable struts (68, 70) encapsulated therebetween.

9. The introducer (10) of claim 8, wherein the inner and outer layers are constructed from a polymeric material.

10. The introducer (10) of claim 7, wherein the plurality of balloon-expandable struts (68) is constructed from a deformable metallic material.

11. The introducer (10) of claim 1, wherein at least a portion of the sheath (56) is comprised of a self-expandable material.

12. The introducer (10) of claim 1, wherein the sheath (56) includes one or more markers (90) comprised of a radiopaque material configured to enable in vivo visualization of the sheath (56).

13. The introducer (10) of claim 1, wherein an inner diameter surface of the sheath (56), an outer diameter surface of the sheath (56), or both is coated with a coating that is operable to lower the coefficient of friction of the surface.

14. The introducer (10) of claim 13, wherein the coating is a hydrophilic coating.

## Patentansprüche

1. Einführungsvorrichtung (10), die so konfiguriert ist, dass sie wenigstens einem chirurgischen Instrument Gefäßzugang während eines perkutanen Verfahrens ermöglicht, wobei die Einführungsvorrichtung (10) Folgendes umfasst:
eine Hülle (56) mit einem proximalen Ende, einem distalen Ende und einem dazwischen verlaufenden Lumen,
**dadurch gekennzeichnet, dass** die Hülle (56) flexibel genug ist, um einen Übergang zwischen einem ersten Blutgefäß und einem zweiten Blutgefäß zu überspannen; und dadurch, dass die Einführungsvorrichtung (10) ferner eine Nabe (54) am proximalen Ende der Hülle (56) umfasst und ein darin angeordnetes Hämostaseventil (60, 61, 62) hat, wobei die Nabe (54) so konfiguriert ist, dass sie außerhalb des Gefäßsystems bleibt, und das Hämostaseventil (60, 61, 62) so konfiguriert ist, dass es den Zugang durch das Lumen der Hülle (56) begrenzt.

2. Einführungsvorrichtung (10) nach Anspruch 1, wobei das Hämostaseventil ein Schlitz (60) in einer Membran (61) oder einer Iris (62) ist.

3. Einführungsvorrichtung (10) nach Anspruch 1, wobei die Hülle (56) von einem ersten Profil, das zum Lenken der Einführungsvorrichtung (10) in das Gefäßsystem konfiguriert ist, zu einem zweiten Profil expandierbar ist, das zum Aufnehmen des chirurgischen Instruments konfiguriert ist.

4. Einführungsvorrichtung (10) nach Anspruch 1, wobei die Flexibilität der Hülle (56) distal zunimmt.

5. Einführungsvorrichtung (10) nach Anspruch 1, wobei wenigstens ein erster Teil (64) der Hülle (56) aus einem ballonexpandierbaren Material besteht.

6. Einführungsvorrichtung (10) nach Anspruch 5, wobei wenigstens ein zweiter Teil (66) der Hülle (56) aus einem selbstexpandierbaren Material besteht, wobei der zweite Teil (66) proximal zum ersten Teil (64) ist.

7. Einführungsvorrichtung (10) nach Anspruch 6, wobei der erste Teil (64) der Hülle (56) mehrere ballonexpandierbare Streben (68) beinhaltet und der zweite Teil (66) mehrere selbstexpandierbare Streben (70) beinhaltet.

8. Einführungsvorrichtung (10) nach Anspruch 7, wobei die Hülle (56) eine innere Schicht und eine äußere Schicht beinhaltet, wobei die mehreren ballonexpandierbaren und selbstexpandierbaren Streben (68, 70) dazwischen eingekapselt sind.

9. Einführungsvorrichtung (10) nach Anspruch 8, wobei die innere und die äußere Schicht aus einem Polymermaterial konstruiert sind.

10. Einführungsvorrichtung (10) nach Anspruch 7, wobei die mehreren ballonexpandierbaren Streben (68) aus einem verformbaren metallischen Material konstruiert sind.

11. Einführungsvorrichtung (10) nach Anspruch 1, wobei wenigstens ein Teil der Hülle (56) aus einem selbstexpandierbaren Material besteht.

12. Einführungsvorrichtung (10) nach Anspruch 1, wobei die Hülle (56) eine oder mehrere Markierungen (90) beinhaltet, die aus einem radiopaken Material bestehen, das so konfiguriert ist, dass eine In-vivo-Visualisierung der Hülle (56) ermöglicht wird.

13. Einführungsvorrichtung (10) nach Anspruch 1, wobei eine Innendurchmesseroberfläche der Hülle (56), eine Außendurchmesseroberfläche der Hülle (56) oder beide mit einer Beschichtung zum Senken des Reibungskoeffizienten der Oberfläche versehen ist/sind.

14. Einführungsvorrichtung (10) nach Anspruch 13, wobei die Beschichtung eine hydrophile Beschichtung ist.

## Revendications

1. Introducteur (10) configuré pour permettre l'accès vasculaire à au moins un instrument chirurgical durant une procédure percutanée, l'introducteur (10) comprenant :
une gaine (56) ayant une extrémité proximale, une extrémité distale et une lumière s'étendant entre elles,
**caractérisé en ce que** la gaine (56) est suffisamment flexible pour couvrir une jonction entre un premier vaisseau sanguin et un deuxième vaisseau sanguin; et **en ce que** l'introducteur (10) comprend en outre un moyeu (54) sur l'extrémité proximale de la gaine (56) et ayant une valve hémostatique (60, 61, 62) disposée dedans, le moyeu (54) étant configuré pour demeurer extérieur à la vascularisation et la valve hémostatique (60, 61, 62) étant configurée pour limiter l'accès à travers la lumière de la gaine (56).

2. Introducteur (10) selon la revendication 1, dans lequel la valve hémostatique est une fente (60) dans une membrane (61) ou un iris (62).

3. Introducteur (10) selon la revendication 1, dans lequel la gaine (56) est dilatable d'un premier profil configuré pour diriger l'introducteur (10) dans la vascularisation à un deuxième profil configuré pour recevoir l'instrument chirurgical.

4. Introducteur (10) selon la revendication 1, dans lequel la flexibilité de la gaine (56) augmente d'une manière distale.

5. Introducteur (10) selon la revendication 1, dans lequel au moins une première partie (64) de la gaine (56) est constituée d'un matériau dilatable à ballonnet.

6. Introducteur (10) selon la revendication 5, dans lequel au moins une deuxième partie (66) de la gaine (56) est constituée d'un matériau auto-dilatable, la deuxième partie (66) étant à proximité de la première partie (64).

7. Introducteur (10) selon la revendication 6, dans lequel la première partie (64) de la gaine (56) comprend une pluralité d'étais dilatables à ballonnet (68) et la deuxième partie (66) comprend une pluralité d'étais auto-dilatables (70).

8. Introducteur (10) selon la revendication 7, dans lequel la gaine (56) comprend une couche interne et une couche externe avec les pluralités d'étais dilatables à ballonnet et auto-dilatables (68, 70) encapsulées entre elles.

9. Introducteur (10) selon la revendication 8, dans lequel les couches interne et externe sont fabriquées en un matériau polymère.

10. Introducteur (10) selon la revendication 7, dans lequel la pluralité d'étais dilatables à ballonnet (68) est fabriquée en un matériau métallique déformable.

11. Introducteur (10) selon la revendication 1, dans lequel au moins une partie de la gaine (56) est constituée d'un matériau auto-dilatable.

12. Introducteur (10) selon la revendication 1, dans lequel la gaine (56) comprend un ou plusieurs marqueurs (90) constitués d'un matériau radio-opaque configuré pour permettre la *visualisation in vivo* de la gaine (56).

13. Introducteur (10) selon la revendication 1, dans lequel une surface du diamètre interne de la gaine (56), une surface du diamètre externe de la gaine (56), ou les deux sont revêtues d'un revêtement qui est opérationnel pour abaisser le coefficient de frottement de la surface.

14. Introducteur (10) selon la revendication 13, dans lequel le revêtement est un revêtement hydrophile.
